# EUROPEAN PATENT APPLICATION

(11) **EP 3 521 340 A1**
(43) Date of publication of application: **07.08.2019**
(21) Application number: 17856888.7
(22) Date of filing: 21.09.2017
(51) Int. Cl.: C08G 83/00, C07F 9/12, C07F 9/40

(54) **A PHTHALONITRILE MONOMER MODIFIED BY ORGANOPHOSPHATE FRAGMENTS, A METHOD FOR OBTAINING SAME, A BINDER BASED THEREON, AND A PREPREG**

(30) Priority: 27.09.2016 RU 2016138335
(71) Applicant: Joint Stock Company Scientific and Production Association "Unichimtek" (JSC PSA "Unichimtek), Moskovskaya obl., 142181 (RU)
(72) Inventor: BULGAKOV, Boris Anatol'evich, Moscow 117447 (RU); BABKIN, Aleksandr Vladimirovich, Moskovskaya obl. 140411 (RU); SULIMOV, Artem Vital'evich, resp. Bashkortostan 450014 (RU); MALAKHO, Artem Petrovich, Moscow 117324 (RU); KEPMAN, Aleksej Valer'evich, Moscow 107143 (RU); AVDEEV, Viktor Vasil'evich, Moscow 117777 (RU)
(74) Representative: Glawe, Delfs, Moll
(86) International application number: PCT/RU2017/000693
(87) International publication number: WO 2018/063030

(57) **Abstract**

This invention relates to the field of organic chemistry, more particularly, to a method of obtaining modified phthalonitriles comprising organophosphorus fragments, binding agents based thereon, as well as products produced with the use thereof, and can be used in a variety of technical fields, for example, in aircraft and automobile manufacturing to produce polymer composite materials. The invention makes it possible to produce phthalonitrile monomers comprising organophosphorus fragments from out of simpler and cheaper reagents, and to obtain them as individual substances, which simplifies the process of synthesis, reduces the glass transition temperature of monomers and increases the thermal oxidation stability of hardened polymers. The phthalonitrile monomer modified with organophosphorus fragments is characterized with the following general formula: 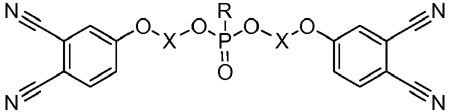 wherein R is a radical selected from the group comprising aryl, alkyl, aryloxy or alkyloxy substituents; and X is a divalent aryl substituent selected from a group comprising phenylenes and naphthylenes. A method of obtaining the modified phthalonitrile monomer according to this invention, a binder, and a prepreg based on the same, are also disclosed.

## Description

### Field of the invention

This invention relates to the field of organic chemistry, more particularly, to a method of obtaining modified phthalonitriles comprising organophosphorus fragments, binding agents based thereon, as well as products produced with the use thereof, and can be used in a variety of technical fields, for example, in aircraft and automobile manufacturing to produce polymer composite materials.

### Prior art

Phthalonitrile-based polymers are characterized by high physical properties and are quite likely to be used as composite material matrices. However, phthalonitrile monomers demonstrate elevated melting/glass transition temperatures, which does not allow the production of PCM (polymer composite materials) by economically efficient methods such as RTM and vacuum infusion.

To eliminate the said flaws, phthalonitrile monomers are subjected to modification.

Thus, patent RU2580927 (ZAO INUMiT) discloses a phthalonitrile monomer modified with organosilicon fragments of the -O-SiR₂-O- group:

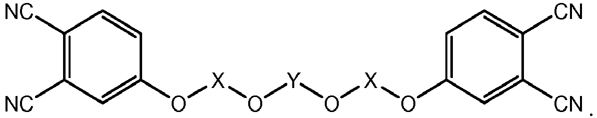

The introduction of such fragments significantly increases the phthalonitrile molecule mobility, which, on the one hand, results in lower glass transition temperatures of the target monomers, and on the other hand, does not lead to a decreased thermal stability of polymers based thereon.

A phthalonitrile monomer is obtained as follows: first, a hydroxyl-containing phthalonitrile is obtained, and then the said hydroxyl-containing phthalonitrile is subjected to interaction with dichlorosilane in an anhydrous aprotic solvent in the presence of a base, with subsequent extraction from the products of interaction of the target product in the form of a phthalonitrile monomer modified with organosilicon fragments.

The above-said patent also discloses a binder and a prepreg based on such modified phthalonitrile.

A phthalonitrile monomer according to the said patent possesses a low glass transition temperature (below 30°C), and, consequently, can be processed in a wide temperature range and form, after hardening, a polymer with a softening temperature of 413 to 434°C.

The closest technical solution to that which is proposed herein is a phthalonitrile modified with organophosphorus fragments (US2011263775, KELLER TEDDY M [US])

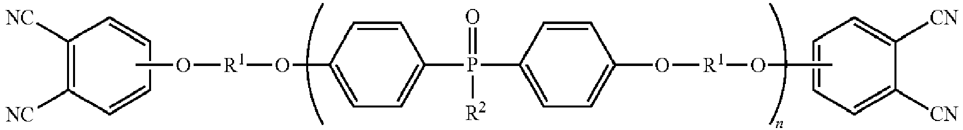

The synthesis of such oligomers includes the reaction of bis(4-fluorophenyl)phenylphosphine oxide, bis(4-fluorophenyl)methylphosphine oxide or a mixture thereof with abundant aromatic diol in the presence of a basic metal carbonate to produce a phthalonitrile oligomer.

The synthesis requires an expensive reagent to be used, namely bis(4-fluorphenyl)phenylphosphine oxide, as well as comonomers bisphenol A or resorcin (see position R1 in the diagram). Moreover, water is produced in the course of the reaction, which needs to be removed from the reaction mixture.

The principal downside of this approach is a complex synthesis of oligomers that includes a number of stages and results in the production of a mixture of products with a variety of chemical makeups. For that reason, the melting of such oligomers takes place in a temperature range (rather than at a certain temperature point), while the resulting molten mass reaches the minimal viscosity values at temperatures significantly exceeding the glass transition temperature of the mixture.

The authors of the prior-art patent cite the following properties: a phthalonitrile with phosphine fragments can withstand continuously high temperatures of from 300 to 375 °C in oxidizing environment, such as air, for a long time without losing strength, the melting temperature of phthalonitrile is from 50 to 100 °C, while polymerization can take place at a temperature above 200 °C.

The objective of this invention is to obtain a phthalonitrile monomer with organophosphorus fragments from simpler and cheaper reagents; to obtain monomers as individual substances, which simplifies the process of synthesis; to reduce the glass transition temperatures of monomers, and increase the thermal oxidation stability of cured polymers.

The said objective is achieved by means of a phthalonitrile monomer modified with organophosphorus fragments having a general formula of 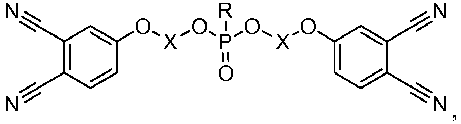
wherein R is a radical selected from the group comprising aryl, alkyl, aryloxy or alkyloxy substituents; and
X is a divalent aryl substituent selected from a group comprising phenylenes and naphthylenes.

The phthalonitrile monomer according to claim 1, wherein R a radical selected from the group consisting of OCH₃, OC₆H₅, OC₁₀H₇, and C₆H₅.

A method of obtaining the phthalonitrile monomer modified with organophosphorus fragments according to claim 1, wherein a reaction is performed between at least one hydroxyl-containing phthalonitrile and a phosphorus-containing substance selected from the group comprising alkyl- or arylphosphoric acid, alkyl- or arylphosphorous acid, alkyl- or arylphosphoric acid anhydrous dichloride and alkyl- or arylphosphorous acid anhydrous dichloride, with subsequent extraction from the reaction products of the target product in the form of a phthalonitrile monomer modified with organophosphorus fragments.

In particular embodiments of the invention, the problem is solved by a method, wherein, as a hydroxyl-containing phthalonitrile, a phthalonitrile selected from the group comprising (4-(2-hydroxyphenoxy)phthalonitrile, 4-(3-hydroxyphenoxy)phthalonitrile and 4-(4-hydroxyphenoxy)phthalonitrile) is used.

Prior to the reaction, the said hydroxyl-containing phthalonitrile is mixed with an acceptable solvent.

The reaction may be performed in the presence of a base.

In particular embodiments of the invention, the extraction of the target product is performed by way of the sequential steps of filtration of the reaction products and obtaining a filtrate, evaporating the filtrate, dissolving the evaporation product, washing the solution with water and extracting the target product therefrom.

The set objective is also achieved by means of a binder that comprises the above described phthalonitrile monomer modified with organophosphorus fragments and aromatic diamine as polymerization initiator.

Aromatic diamine content may be as high as 20 % by weight.

In particular embodiments of the invention the binder can be used as such to obtain polymer composite materials by infusion technology.

The set objective is also achieved by means of a prepreg made of the binder and a reinforcing element.

The prepreg may contain, as a reinforcing element, an element selected from the group consisting of unidirectional carbon tapes, carbon fabrics, fiberglass fabrics, unidirectional fiberglass tapes, chopped carbon fibers, and chopped fiberglass fibers.

The essence of the invention consists in the following.

The introduction of organophosphorus fragments as per this invention does not only result in the lower glass transition temperature of the target products due to the flexibility of phosphate and phosphonate fragments, but also in the improved thermal oxidation stability of polymers based on phthalonitriles modified with organophosphorus fragments.

The claimed phthalonitrile monomer with organophosphorus fragments is obtained by the following reactions proceeding according to Charts 1 and 2:

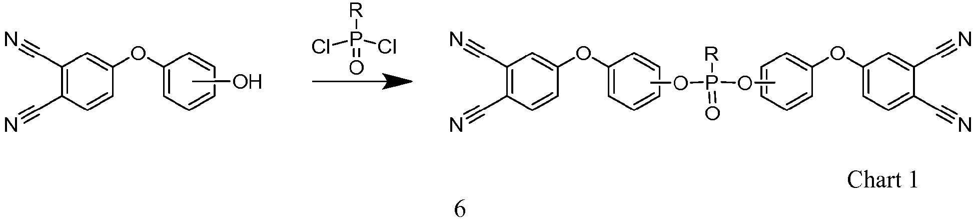

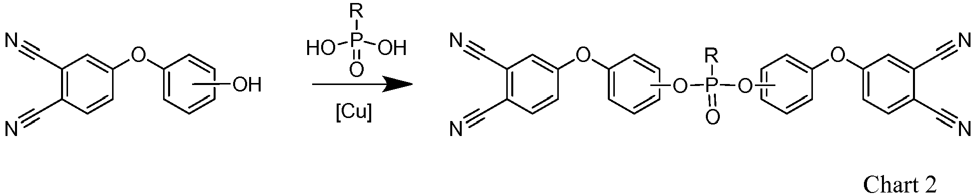

Hydroxyphenoxy-phthalonitrile (prepared from 4-nitrophthalonitrile and one of the three dihydroxybenzenes) reacts with a phosphoric or phosphorous acid derivative, which may be:
1) arylphosphoric acid anhydrous dichloride, alkylphosphoric acid anhydrous dichloride, arylphosphorous acid anhydrous dichloride, alkylphosphorous acid anhydrous dichloride (Chart 1);
2) alkyl- or arylphosphoric acid, alkyl- or arylphosphorous acid (Chart 2).

As a result of the reaction, phthalonitrile modified with organophosphorus fragments will comprise phosphoric or phosphorous acid ethers, while in the prior-art solution (US2011263775), which also treats the preparation of a phthalonitrile (oligomer) modified with organophosphorus fragments, phosphorus is introduced as a triphenylphosphine oxide derivative, meaning that the bonding with phthalonitrile groups and phosphorus takes place via C-P, and phosphorus oxidation state in the described compounds is equal to (-1).

In our case, the bonding occurs through the formation of phosphoric or phosphorous acid ethers, that is an O-P bond, meaning that phosphorus oxidation state is +5 or +3, respectively.

Another fundamental difference from the prior-art phthalonitrile consists in the fact that we, as per Charts 1 and 2, obtain an individual substance, while US2011263775 yields a mixture of oligomers.

The invention operates as follows.

A series of phthalonitrile monomers comprising different organophosphorus fragments was synthesized. 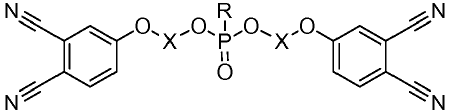 wherein R = aryl, alkyl, aryloxy or alkyloxy substituents, for example: OCH₃, OC₃H₇, OC₃H₃, OC₆H₅, OC₁₀H₇, CH₃, C₃H₇, C₃H₃, C₆H₅, C₁₀H₇, while substituent X is a divalent aryl substituent selected from phenylenes or naphthylenes, for example 1,2-phenylene, 1,3-phenylene, 1,4-phenylene, naphthylene.

For this purpose, a reaction was conducted between hydroxyl-containing phthalonitriles and phosphorus-containing substances, for example, anhydrous dichlorides of phosphorus-containing acids. 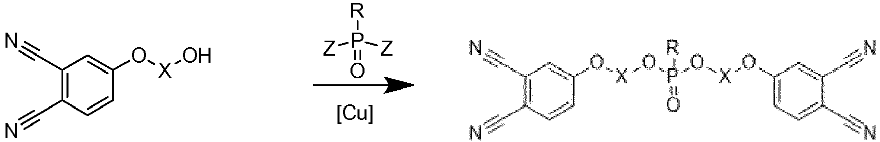 wherein X is an aryl or alkyl divalent fragment, Z is a halogen or a hydroxyl.

Any hydroxyl-containing phthalonitrile may be used for the purposes of this invention, for example, 4-hydroxyphthalonitrile; 4-(2-hydroxyphenoxy)phthalonitrile; 4-(3-hydroxyphenoxy)phthalonitrile; 4-(4-hydroxyphenoxy)phthalonitrile; 4-((6-hydroxynaphthalene-2-yl)oxy)phthalonitrile; 4-((5-hydroxynaphthalene-1-yl)oxy)phthalonitrile; 4-(4-(1,1,1,3,3,3-hexafluor-2-(4-hydroxyphenyl)propane-2-yl)phenoxy)phthalonitrile; 4-(4-(hydroxymethyl)phenoxy)phthalonitrile; 4-(3-(hydroxymethyl)phenoxy)phthalonitrile; 4-(2-(hydroxymethyl)phenoxy)phthalonitrile. This list in not exhaustive.

In this case, it is not the composition of hydroxyl-containing phthalonitrile as such, but rather the presence of hydroxyl bond in it which allows obtaining an O-P-O bond bridge in the process of the reaction, which in turn reduces the glass transition temperature of the monomer and increases the thermal oxidation stability of the polymer based thereon.

Hydroxyl-containing phthalonitriles for the synthesis of phosphorus-containing monomers may be lawfully bought or synthesized according to publicly disclosed methodologies, for example, 4-(2-hydroxyphenoxy)phthalonitrile can be synthesized according to the method described in the article [D. González-Rodríguez, E. Carbonell, C. A. Castellanos, D. M. Guldi, T. Torres. //J. AM. CHEM. SOC. 2010, 132, 16488-16500]*;* 4-(3-hydroxyphenoxy)phthalonitrile - according to the method described in [Lyubimtsev, A.; Vagin, S.; Syrbu, S.; Hanack, M. // Eur. J. Org. Chem. 2007, 2007 (12), 2000-2005], while the preparation of 4-(4-hydroxyphenoxy)phthalonitrile was disclosed in the article [O. Tsaryova, A. Semioshkin, D. Wöhrle, V. I. Bregadze // J. Porphyr Phthalocyanines 2005 09:04, 268-274].

In particular embodiments of the invention, it was specifically those available and readily producible hydroxyl-containing phthalonitriles namely (4-(2-hydroxyphenoxy)phthalonitrile, 4-(3-hydroxyphenoxy)phthalonitrile and 4-(4-hydroxyphenoxy)phthalonitrile) that were used, which made it possible to reduce the cost of the process.

In the capacity of phosphorus-containing substances, as indicated above, a wide range of substances may also be used, such as anhydrous dichlorides of alkyl- or arylphosphoric acid and anhydrous dichlorides of alkyl- or arylphosphorous acid (Chart 1).

Besides, the formation reaction may proceed directly with alkyl- or arylphosphoric acid, or alkyl- or arylphosphorous acid (Chart 2).

Prior to reaction, hydroxyl-containing phthalonitrile monomers were dissolved, which ensured better interaction of hydroxyl-containing phthalonitriles with phosphorus-containing substances. Any acceptable solvents can be used to dissolve hydroxyl-containing phthalonitrile monomers, for example, dichloromethane, chloroform, benzene, toluene, xylene, benzene chloride, dimethyl formamide, dimethyl sulfoxide, tetrahydrofuran, dioxane, acetonitrile, acetone, diethyl ether, dibutyl ether and others.

A reaction between hydroxyl-containing phthalonitriles and anhydrous dichlorides of phosphorus-containing acids can proceed both without a base and in the presence of a base, the base being, for example: triethylamine, ethylene diamine, aniline, diamino benzenes, pyridine, chinoline, diazabicyclooctane, diazabicycloundecene, and sodium, potassium, calcium and strontium carbonates.

A reaction between hydroxyl-containing phthalonitriles and phosphorus-containing acids proceeds in the presence of copper catalysts, such as CuCl, CuBr, CuI, CuCl₂, CuBr₂, Cu(OAc)₂, Cu₂O, CuO, Cu (powder), and in the presence of such bases as: NaHCO₃, Na₂CO₃, KOH, K₃PO₄, t-BuONa, AcOK, triethylamine and tetrachloromethane in such solvents as dichloromethane, trichloromethane, dichloroethane, benzene, toluene and others.

The extraction of the target product from the reaction products does not present any special difficulties. As a rule, it is sufficient to wash the post-reaction mixture with water until the water becomes weakly acidic. After that the organic layer is dried and solvent is removed therefrom. Such method is used where the reaction with anhydrous dichloride of phosphorus-containing acid is conducted without a base.

In the presence of a base, the target product is extracted according to the following: the post-reaction mixture is filtered to obtain salts of hydrochloric acid, evaporated to a minimal volume, dissolved in a solvent and washed with water. Then the organic layer is dried and solvent removed therefrom.

At room temperature, the obtained phthalonitrile monomer modified with organophosphorus fragments has the form of a glassy mass.

Phthalonitriles modified with organophosphorus fragments may be cured in the presence of such aromatic diamines as diamine R, diamino benzenes, 3,3'-diaminodiphenyl sulfone, 4,4'-diaminodiphenyl sulfone; or diphenols: bisphenol A, bisphenol C and others.

A phthalonitrile monomer modified with organophosphorus fragments can be used to prepare a binder that can be used, in turn, to produce both prepregs and composite materials; it is particularly well-suited for the production of composites by the RTM and vacuum infusion methods.

The binder, besides phthalonitrile modified with organophosphorus fragments, additionally comprises an aromatic diamine as a polymerization initiator. In the best embodiments of the invention the amount of aromatic diamine does not exceed 20% by weight.

One can use, as polymerization initiators, such aromatic diamines as, for example, diamine R, diaminobenzenes, 3,3'-diaminodiphenyl sulfone, 4,4'-diaminodiphenyl sulfone; or diphenols: bisphenol A, bisphenol C and others.

The prepreg was prepared by saturation with the obtained binder of a reinforcing filler, which can be selected from a wide range of materials from reinforcing fibers to fabrics made of such fibers.

An important feature of the claimed method of producing modified phthalonitrile monomer is the fact that the process runs in one stage without any intermediary reaction products being discharged, while the process according to the prior art process (US2011263775) runs in such a way that water is discharged in the course of the reaction which needs to be removed from the reaction mixture, and the product is a mixture of a variety of substances with different chemical makeups. Due to this circumstance, the melting of such oligomers occurs in a temperature range rather than at a certain temperature point, and the obtained melt reaches minimal viscosity values at temperatures greatly exceeding the glass transition temperature of the mixture.

It is also worth noting that the synthesis of oligomers under Application US2011263775 assumes the use of an expensive reagent namely bis(4-fluorphenyl)phenylphosphine oxide, as well as comonomers bisphenol A or resorcin, while the process according to this invention is devoid of this drawback.

The invention is illustrated by the following examples.

### Example 1.

Preparation of phthalonitrile bis(3-(3,4-dicyanophenoxy)phenyl)phenyl phosphate modified with organophosphorus fragments. 4.5 grams of phenyl dichlorophosphate were added dropwise to a suspension of 9.44 grams of 4-(3-hydroxyphenoxy)phthalonitrile in 50 ml of toluene. The mix was stirred for 24 hours until hydrogen chloride stopped to be emitted. After that, the reaction mixture was washed with water (5×25 ml) until the water became weakly acidic. After that, the organic layer was dried over anhydrous Na₂SO₄ and the solvent was removed from the flask. The target substance was obtained in the form of yellowish glassy mass. Yield - 90%.

### Example 2.

Preparation of phthalonitrile bis(3-(3,4-dicyanophenoxy)phenyl) phenyl phosphonate modified with organophosphorus fragments. 4.15 grams of phenyl dichlorophosphate were added dropwise to a suspension of 9.44 grams of 4-(3-hydroxyphenoxy)phthalonitrile in 50 ml of chlorobenzene. The mix was stirred for 12 hours in the presence of 4.3 g of dry triethylamine as a base. After that, the reaction mix was filtered on a glass filter from triethylammonium hydrochloride, and washed with toluene (3 × 10 ml). Then the mixture was washed with water (3 × 25 ml), dried over anhydrous Na₂SO₄ and the solvent was removed from the flask. The target substance was obtained in the form of yellowish glassy mass. Yield - 95%.

### Example 3.

Preparation of phthalonitrile bis(3-(3,4-dicyanophenoxy)phenyl)naphthyl phosphate modified with organophosphorus fragments. 5.6 grams of naphthyl dichlorophosphate were added dropwise to a suspension of 9.44 grams of 4-(3-hydroxyphenoxy)phthalonitrile in 50 ml of chloroform. The mix was stirred for 6 hours in the presence of 10.7 g strontium carbonate as a base. After that, the reaction mix was filtered on a glass filter from inorganic substances, and washed with chloroform (3 × 10 ml). After that, the mixture was washed with water (3 × 25 ml), dried over anhydrous Na₂SO₄ and the solvent was removed from the flask. The target substance was obtained in the form of yellowish glassy mass. Yield - 77%.

### Example 4.

Preparation of phthalonitrile bis(3-(3,4-dicyanophenoxy)phenyl)isopropyl phosphate modified with organophosphorus fragments. 3.8 grams of isopropyl dichlorophosphate was added dropwise to a suspension of 9.44 grams of 4-(2-hydroxyphenoxy)phthalonitrile in 35 ml of dry dimethyl formamide. The mix was stirred for 4 hours in the presence of 3.4 g of pyridine as a base. After that, the reaction mix was filtered on a glass filter from pyridine hydrochloride, evaporated to a minimal volume, dissolved in methylene chloride and washed with water (3 × 20 ml). After that, the organic layer was dried over anhydrous Na₂SO₄ and the solvent removed from the flask. The target substance was obtained in the form of yellowish glassy mass. Yield - 92%.

### Example 5.

Preparation of phthalonitrile bis(4-(3,4-dicyanophenoxy)phenyl)phenyl phosphate modified with organophosphorus fragments. A mixture of phenylphosphoric acid (5 mmol), 4-(3-hydroxyphenoxy)phthalonitrile (10 mmol), Na₂CO₃ (10 mmol), CCl₄ (20 mmol), Et₃N (5 mmol) and powdered copper (2.5 mmol) in 30 ml of CH₂Cl₂ was stirred at 100 °C in the air for 12 hours. After that, the reaction mixture was washed with water and dried over anhydrous sodium sulfate, with the subsequent removal of the solvent on a rotary evaporator. The target monomer bis(3-(3,4-dicyanophenoxy)phenyl)phenyl phosphate was obtained in the form of brown glassy mass.

### Example 6.

Preparation of a binder from phthalonitrile modified with organophosphorus fragments, namely from bis(3-(3,4-dicyanophenoxy)phenyl)phenyl phosphate obtained according to Example 1. The monomer (0.5 g) and a hardener (bis-aminophenoxybenzene (diamine P), 0.02 g) were placed in a glass vial; the components were melted together at 120-180 °C.

### Example 7.

Preparation of a binder from bis(4-(3,4-dicyanophenoxy)phenyl)phenyl phosphate modified with organophosphorus fragments obtained according to Example 5. The monomer (0.5 g) and a hardener (3,3-diaminodiphenyl sulfone, 0.015 g) were placed in a glass vial; the components were melted together at 120-180 °C in vacuum.

### Example 8.

Hardening of the binder prepared according to Examples 6 and 7. The vial with a binder prepared according to Examples 6 or 7 was connected to a pump, vacuumed, and the content heated to melting at 120-180 °C. After that the molten mass was shaken in vacuum to remove the dissolved gases, then the vial was filled with argon and the obtained binder hardened stepwise with exposures for 8 hours at 250 °C, for 6 hours at 315 °C, and for 8 hours at 375 °C.

### Example 9.

After that, a prepreg was prepared from the above-described binders.

For that purpose, one of the binders consisting of modified phthalonitriles according to Examples 6 and 7 was fed onto the rollers. A binder film with a preset thickness calculated as 40 mass % of the surface density of the final prepreg was transferred onto siliconized paper by means of the rollers. A binder film with a preset thickness calculated as 40 mass % of the surface density of the final prepreg was transferred onto siliconized paper by means of the rollers.

The obtained prepreg was rolled up into rolls.

The prepreg possesses stickiness at room temperature.

The binder made from modified phthalonitriles as per Examples 6 and 7 was tested as a binding agent for the infusion technology. As polymerization initiators, the following diamines were used: diamine P in the amount of 4% by weight, and 3,3'-diaminodiphenyl sulfone in the amount of 3%.

Eight layers of unidirectional carbon tape with a density of 200 g/m² were laid in vacuum infusion molds, then air was removed, and the binder pre-heated to 100°C was fed at 110°C inside the mold. Then the product was cured in a stepwise manner with exposures at 200°C, 250°C, 300°C, 350°C, and 375°C.

These and other examples of embodiments of the invention, as well as the properties of modified phthalonitrile monomers, are shown in Tables 1 and 2.

As follows from the presented data, the introduction of an organophosphorus modifier into the structure of the molecule of phthalonitrile monomer results in a reduction of glass transition temperature of the monomer and increases its thermal oxidation stability, which allows using such monomers to produce a binder for PCM molding by prepreg technology, as well as by the methods of vacuum infusion and polymer injection into the mold (RTM).

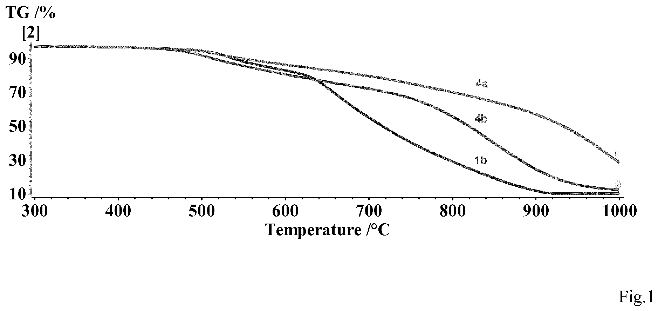

Fig. 1 shows the relationship between the mass loss of a hardened phthalonitrile and the heating temperature in the air for the oligomer according to this invention (4a), the monomer according to US4587325 (4b) and the monomer according to patent RU2580927 (1b).

As follows from the data in Fig. 1, the phthalonitrile monomer as per this invention withstands heating to temperatures above 500°C, with gradual deterioration of its properties under further heating; the phthalonitrile according to RU2580927 withstands temperatures around 420°C, with a dramatic increase of mass loss under further heating. A similar behavior is demonstrated by the phthalonitrile oligomer according to US2011263775.

**Table 1**

| Monomer | R | X | Properties of obtained monomer |
|---|---|---|---|
| | | | Glass transition temperature Tgt, °C |
| 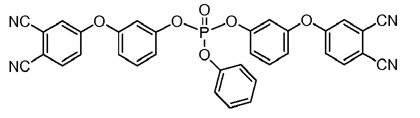 | OC₆H₅ | 1,3 phenylene | 22 |
| 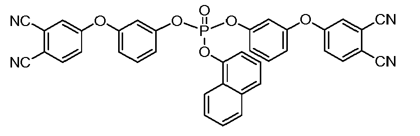 | OC₁₀H₇ | 1,3 phenylene | 58 |
| 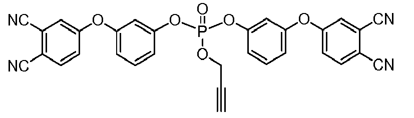 | OC₃H₃ | 1,3 phenylene | 4 |
| 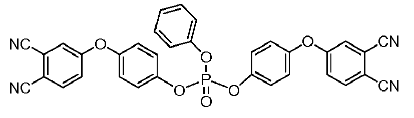 | OC₆H₅ | 1,3 phenylene | 50 |
| 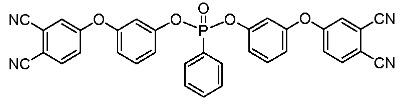 | C₆H₅ | 1,3 phenylene | 42 |

| Monomer | Hardened monomer (binder)* properties | | | | |
|---|---|---|---|---|---|
| | Softening temperature, T_{soft} (heat resistance), ° C | Start of decomposition in argon, (loss of 5% of mass), T_{5%}, °C | Carbon residue, % | Start of decomposition in argon, (loss of 5% of mass), T_{5%}, °C | Ash residue, % |
| 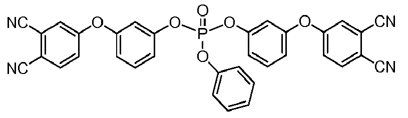 | 455 | 524 | 80 | 523 | 29 |
| 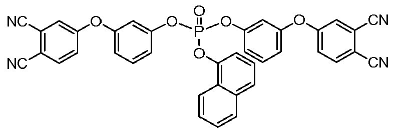 | - | 512 | 77 | 516 | 6 |
| 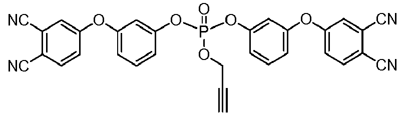 | 441 | 518 | 80 | 514 | 49 |
| 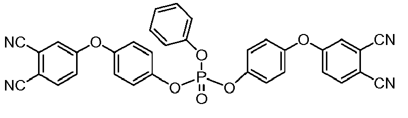 | - | 514 | 78 | 501 | 37 |
| 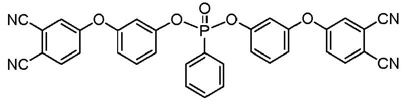 | - | 498 | 72 | 494 | 15 |

| | | | | | |
|---|---|---|---|---|---|
| *Binders prepared to Examples 6 and 7 and hardened according to Example 8 in the presence of 4% diamine P (PPD). | | | | | |

## Claims

1. A phthalonitrile monomer modified with organophosphorus fragments having a general formula of 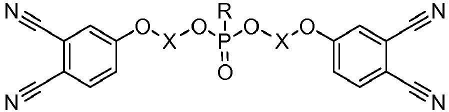
wherein R is a radical selected from the group comprising aryl, alkyl, aryloxy or alkyloxy substituents; and
X is a divalent aryl substituent selected from the group comprising phenylenes and naphthylenes.

2. The phthalonitrile monomer according to claim 1, wherein R is a radical selected from the group comprising OCH₃, OC₆H₅, OC₁₀H₇, C₆H₅.

3. A method of obtaining the phthalonitrile monomer modified with organophosphorus fragments according to claim 1, wherein a reaction is performed between at least one hydroxyl-containing phthalonitrile and a phosphorus-containing substance selected from the group comprising alkyl- or arylphosphoric acid, alkyl- or arylphosphorous acid, anhydrous dichloride of alkyl- or arylphosphoric acid and anhydrous dichloride of alkyl- or arylphosphorous acid, with subsequent extraction from the reaction products of the target product in the form of a phthalonitrile monomer modified with organophosphorus fragments.

4. The method according to claim 3, **characterized in that** a phthalonitrile selected from the group comprising (4-(2-hydroxyphenoxy)phthalonitrile, 4-(3-hydroxyphenoxy)phthalonitrile and 4-(4-hydroxyphenoxy)phthalonitrile) is used as a hydroxyl-containing phthalonitrile.

5. The method according to claim 3, **characterized in that** prior to reaction, the said hydroxyl-containing phthalonitrile is mixed with an acceptable solvent.

6. The method according to claim 3, **characterized in that** the reaction is performed in the presence of a base.

7. The method according to claim 3, **characterized in that** the extraction of the target product is performed by way of sequential stages of filtering of the reaction products and obtaining a filtrate, evaporating the filtrate, dissolving the evaporation product, washing the solution with water and extracting the target product therefrom.

8. A binder **characterized in that** it comprises the phthalonitrile monomer modified with organophosphorus fragments according to claims 1 or 2 and an aromatic diamine as a polymerization initiator.

9. The binder according to claim 8, **characterized in that** it comprises such aromatic diamine in an amount of up to 20% by weight.

10. The binder according to claim 8, **characterized in that** it is used as a binder to obtain polymer composite materials by infusion technology.

11. A prepreg **characterized in that** it is made of the binder according to any of claim 8 to 10 and a reinforcing element.

12. The prepreg according to claim 11, **characterized in that** it comprises, as a reinforcing element, an element selected from the group comprising unidirectional carbon tapes, carbon fabrics, fiberglass fabrics, unidirectional fiberglass tapes, chopped carbon fibers, and chopped fiberglass fibers.
